# EUROPEAN PATENT SPECIFICATION

(11) **EP 2 054 729 B1**
(45) Date of publication and mention of the grant of the patent: **30.09.2009**
(21) Application number: 06778361.3
(22) Date of filing: 25.08.2006
(51) Int. Cl.: G01N 33/68, G01N 33/543

(54) **PROCESS AND APPARATUS FOR ION CHANNEL CHARACTERIZATION**
VERFAHREN UND VORRICHTUNG ZUR CHARAKTERISIERUNG VON IONENKANÄLEN
PROCÉDÉ ET APPAREIL POUR LA CARACTÉRISATION D'UN CANAL D'IONS

(43) Date of publication of application: 06.05.2009
(73) Proprietor: Universität Hannover, 30167 Hannover (DE)
(72) Inventor: ZEILINGER, Carsten, 30167 Hannover (DE)
(74) Representative: Taruttis, Stefan Georg
(86) International application number: PCT/EP2006/065704
(87) International publication number: WO 2008/022658

(56) References cited:
- WO-A-01/68922
- WO-A-20/05069008
- DE-A1-102004 062 273
- VOETS THOMAS ET AL: "The principle of temperature-dependent gating in cold- and heat-sensitive TRP channels." NATURE. 12 AUG 2004, vol. 430, no. 7001, 12 August 2004 (2004-08-12), pages 748-754, XP002412896 ISSN: 1476-4687 cited in the application
- GAVVA NARENDER R ET AL: "AMG 9810 [(E)-3-(4-t-butylphenyl)-N-(2,3-dihydroben zo[b][1,4] dioxin-6-yl)acrylamide], a novel vanilloid receptor 1 (TRPV1) antagonist with antihyperalgesic properties." THE JOURNAL OF PHARMACOLOGY AND EXPERIMENTAL THERAPEUTICS. APR 2005, vol. 313, no. 1, April 2005 (2005-04), pages 474-484, XP002413335 ISSN: 0022-3565
- DENYER J ET AL: "HTS APPROACHES TO VOLTAGE-GATED ION CHANNEL DRUG DISCOVERY" DDT - DRUG DISCOVERY TODAY, ELSEVIER SCIENCE LTD, GB, vol. 3, no. 7, 1998, pages 323-332, XP000910805 ISSN: 1359-6446
- RODRÍGUEZ B M ET AL: "Voltage gating of Shaker K+ channels. The effect of temperature on ionic and gating currents." THE JOURNAL OF GENERAL PHYSIOLOGY. AUG 1998, vol. 112, no. 2, August 1998 (1998-08), pages 223-242, XP002412897 ISSN: 0022-1295 cited in the application
- RICHARD G: "Connexins: a connection with the skin." EXPERIMENTAL DERMATOLOGY. APR 2000, vol. 9, no. 2, April 2000 (2000-04), pages 77-96, XP002413336 ISSN: 0906-6705

## Description

The present invention relates to a process and an apparatus for performing the process, suitable for ion channel characterisation, especially to the determination of ion channel activity and the impact of effector compounds onto the ion channel activity.

Ion channels are membrane proteins that regulate the permeation of ions, e.g. of sodium and/or potassium ions and/or of peptides or nucleic acids across cellular membranes. Ion channels regulate permeation of ions by changing their gating between a closed state and an open state, which gating is usually dependent upon the presence or absence of membrane potential activator or inhibitor substances or conditions, acting as stimuli for the ion channel activity.

### State of the art

To-date , a standard analytical method for measuring ion channel activity, i.e. the gating of ion permeation, is the patch clamp technique, using whole cells for the measurement of the variation of the total current across the entire surface of the cell membrane. In general, patch clamp is a technique that requires a skilled experimentor for the micro-manipulation of a glass pipette to be attached to the outer surface a single cell under a microscope and rupturing the membrane in the area of the aperture of the pipette. Using this access to the interior of the cell, voltage and/or current measurements can be made in relation to a second electrode arranged in the surrounding medium, for one cell at a time.

Fertig et al. (Biophysical Journal 3056 -3062, 2002) describe the development of a quartz glass plate having a thickness of 200 µm with a defined aperture of about 1 µm. This aperture is said to be suitable for replacing glass pipettes that were used for patch clamp techniques so far, because cells could sealingly be positioned over the aperture.

Voets et al. (Nature, 748-754, 2004) describe mammalian temperature sensitive ion channels, classified as belonging to the group of transient receptor potential superfamily of cation channels to have a temperature sensing activity which is tightly linked to voltage dependent gating, both in a cold sensitive channel and a heat sensitive channel. Both types of channels could be shown to be activated by depolarisation. Temperature changes result in graded shifts of the voltage dependent activation. For analysis, whole mammalian cells were used in one-cell patch clamp experiments.

The methods available for measuring the effect of a stimulating compound, for example an inhibitor or activator, upon the gating activity of an ion channel in the case of patch clamp techniques require high precision handling of cells and are therefore not suitable for automatisation. For automatisation, reliability and reproduceability of results, patch clamp techniques, even when using a defined aperture in a dielectric substrate for attaching the cell to be measured, suffer from the difficulties related to the formation of a tight seal between the cell and the aperture and, as a consequence can show a large variation of measurement results.

Rodriguez ct al. (J. Gen. Physiol. 223-242, 1998) generally cite previous articles, e.g. Hodgkin and Huxley 1952, in that temperature is known to affect the activation kinetics of ion channels. While investigating the voltage gating of Shaker K⁺ channels in currency measurements, it was found that for currency measurements, a minimum temperature was necessary for identifying ion permeation activity of the channel protein because at lower temperatures, the Shaker K⁺ channel was found to be closed. In the experiments, voltage dependency of the current was determined. However, at the low voltages used, the activation of the channel protein was to low for resolving the activation kinetics.

Parfenova et al. (JBC Papers in Press, 1-19, May 25, 2006) describe that the calcium gated potassium channel MthK was open to potassium uptake even without calcium²⁺ when measured at 37 °C.

Gavva et al. (The Journal of Pharmacology and Experimental Therapeutics, 474-484 (2005)) describe an antagonist for the vanilloid receptor 1 (TRPV1). Using whole-cell patch-clamp technique and activation of gating activity by capsaicin, protons and heat, antagonist assays were performed in cultivated rat dorsal root ganglion cells expressing the cation channel receptor.

WO 2005/069008 A1 describes a method for measuring the activity of ion channels at low temperatures, e.g. below 10 °C. For detection of gating activity, potential - sensitive fluorescence dyes are used, which dyes can enter the cell upon depolarisation of the resting membrane potential, leading to an increase in fluorescence activity in dependence on gating activity of ion channel protein.

WO 01/68922 A1 describes the measurement of gating activity of calcium dependent ion channels expressed by cells in the presence of an intracellular chelator of calcium by comparing the signal measured for a first aliquot exposed to a potential agonist with the signal obtained for a second aliquot in the absence of the agonist or inhibitor. In the assay, an intracellular chelator of calcium is used for delaying the signal generated by the calcium sensitive ion channel.

DE 10 2004 062 273 A1 discloses a process for detecting the effect of a putative effector agent on an insect calcium ion channel. In the assay, a eucaryotic host cell expressing insect CNG1 and calcium sensitive luciferase binding apoprotein is used in the presence of a calcium chelator. For detection of the effect of the putative effector agent, this putative effector agent is added to the recombinant host cells, followed by adding an excess of calcium ions and measuring the level of intracellular calcium.

Denyer et al. (Science 323-332 (1989)) generally describes the use of calcium dependent fluorescent dyes for measuring gating activity of calcium dependent ion channels contained in cells.

Richard (Experimental Dermatology, 77-96 (2000)) describes the voltage dependency, calcium and pH sensitivity of connexins, which are gap junctional channels, and the pleiotrophic effect of mutations in the connexin in humans.

### Objects of the invention

In view of the physiological and medical importance of ion channels as drug targets, it is desirable to provide a method, and the use of an apparatus for performing the method, for reliable measurement of ion channel gating activity in response to compounds of interest, e.g. for screening effector compounds like inhibitors and activators.

It is a preferred object of the present invention to provide such a method and the use of an apparatus, preferably in an embodiment suitable for automatisation for use in screening methods, preferably for use in high throughput screening methods.

### General description of the invention

The present invention achieves the above-mentioned objects by providing a method and the use of an apparatus for measuring the gating activity of ion channels which are comprised in lipid membrane vesicles. For detection, the generation or change of an optically detectable signal, e.g. of a dye like a colorimetrically detectable or a fluorescent compound, in dependency from the gating activity of the ion channel is used. Therefore, gating is measureable as a change, e.g. an increase, of the optically detectable signal dependent on the permeation of an optically detectable compound or of ions from the vesicle into the surrounding medium upon activation of the open state of the ion channel.

For measurement of the effect of a compound of interest, herein also termed effector, onto the gating activity of the ion channel, the effector is to be contacted with the ion channel, before, during or following generation of the ion channel containing lipid membrane vesicles.

As a characterizing feature of the invention, the gating activity of permeation through the ion channel is initiated or triggered by applying a temperature shift, preferably as a rise in temperature from a low temperature of about 0 to 5 ° to a temperature of 15, 20, 25, 30, 37 or 42 °C. For generating the temperature shift, a thermostat is present in or surrounding the ion channel containing vesicle preparation, preferably using incremental changes in temperature.

Alternatively, the gating activity of the ion channel is induced by high energy laser pulses, e.g. having a wavelength of 266 to 1800 nm, and/or by ultrasonic vibration.

For detection of ion channel gating activity, it is preferred to generate lipid membrane vesicles having the ion channel integrated into the lipid membrane and containing in the inner volume an ion species transported or permeated through the ion channel. An ion species transported or permeated through the ion channel can e.g. be alkaline or alkaline earth ions, for example sodium ions, potassium ions, magnesium ions, and/or calcium ions. Permeation of the ion species into the surrounding medium, which is depleted of that ion species, therefore leads to an increase in the concentration of the permeated ion species. Accordingly, the increase in concentration of the ion species permeated through the ion channels can be detected, for example by determination of electric parameters like conductivity or electric capacity. However, it is preferred to detect the increase in concentration of the ion species by colorimetric of fluorescence measurements, e.g. by a change of colour or fluorescence of a suitable dye, preferably a fluorescent dye, present in the surrounding medium.

Suitable dyes, specific for detection of an increase in the concentration of potassium ions can be selected from the group comprising PBFI fluorescent dyes. For the detection of sodium ions, SBFI dyes, CoraNa Green Na⁺ indicator, CoraNa Red Na⁺ indicator or Sodium Green (available from Molecular Probes) can be used. PBFI and SBFI, both available from Molecular Probes, USA, and Invitrogen, Germany, contain benzofuranyl fluorophores linked to a crown ether chelator. As the cavity size of the crown ether confers selectivity for sodium and potassium, respectively, binding of the specific ion increases the dye's fluorescence quantum yield and its excitation peak narrows and its excitation maximum shifts to shorter wavelenghts. Accordingly, presence of the ions can specifically be detected as a change in the ratio of fluorescence intensities excited at 340/380 nm. Using emission ration detection, an excitation at 340 nm can be used with a detection at 410/590 nm. Sodium Green contains two 2',7'-dichlorofluorescein dyes linked to the nitrogen atoms of a crown ether having a cavity size specific for sodium ions.

For the detection of calcium and/or magnesium ions Arsenazo III dyes, and for the detection of chloride ions SPQ dyes can be used as components of the medium surrounding the lipid membrane vesicles.

Within the context of the invention, permeation mediated by ion channels also includes the transport of protein, peptide and/or nucleic acids, e.g. hormones or antigen, and, accordingly, these compounds are included by the reference to ions transported by permeation. Examples for ion channels suitable for permeation of protein, peptides and/or nucleic acid are comprised in the group of the connexin family, mediating e.g. intracellular or intercellular transport, e.g. for signalling or cross-presentation of antigen between different cells. Exemplary connexins include Cx43. For use in the present invention, it is preferred to label protein, peptides and/or nucleic acids for detection of permeation from the inner volume of the vesicle into the surrounding medium, e.g. by covalently attaching a detectable label, for example selected from the group comprising dyes, preferably fluorescent dyes, e.g. Lucifer Yellow (LY).

In the process using an ion channel, the gating activity for permeation is blocked by the presence of a specific blocking agent. The presence of the blocking agent is used during the preparation of the lipid membrane vesicles containing the ion channel in the lipid membrane, e.g. by incubating the ion channel protein in the blocking agent before, during or following incorporation into the vesicles.

As an example for a blocking agent active on ion permeating ion channels, calcium²⁺ ions can be used, acting as inhibitors for permeation of ions for certain ion channels. In this embodiment, prior to the application of the temperature change, the blocking agent is to be separated or inactivated, e.g. by masking, complexing or by dilution under a threshold to create a concentration of the blocking agent that does not significantly influence the gating activity of the ion channel. In the case of calcium²⁺ ions as the blocking agent for ion channels, complexing with EDTA can be used to effectively remove the blocking agent.

As the presence of a blocking agent blocks permeation by the ion channel also at temperatures above the permissive temperature, at which in the absence of the blocking agent permeation would occur, in a further embodiment of the invention, effective removal of the blocking agent from the vesicle preparation at or above the permissive temperature can be used for triggering permeation. In this embodiment, the temperature at which permeation occurs is given, but permeation is blocked by the blocking agent. Effective removal of the blocking agent therefore unblocks permeation, serving as a trigger for gating activity.

The process of the invention allows to identify the effect of an effector compound on the gating activity of an ion channel in a precise manner because a correlation of the temperature attained during initiation the gating activity in the presence of the effector to the temperature attained for initiating gating activity in a control assay without presence of the effector can be used as a measure for an influence of the effector to the ion channel. Therefore, effector compounds can be screened by the method of the invention in respect of their activating or inhibitory effect on the ion channel, e.g. both quantitatively and qualitatively.

Because the process and the apparatus necessary for performing the process essentially require the handling of liquid preparations only and the manipulation, i.e. the change of temperature of the components during the assaying process without physical manipulation, e.g. by withdrawal or introduction of thermal energy, preferably using a laser, and/or by application of ultrasonic vibration, the process and apparatus is perfectly suitable for automatisation. The embodiment in which effective removal of a blocking agent at a temperature allowing permeation by addition of an agent complexing or masking the blocking agent is used for triggering permeation, e.g. EDTA in the case of bivalent cations, is also suitable for automatisation as only standard liquid handling is required.

State of art patch clamp techniques, even if an aperture for attaching the cell under investigation is provided on a sheet-like substrate, always require precise mechanic or fluid-dynamic manipulation of cells, namely the localization of the cell above the apperture with the necessity to form an electrically insulating seal to the substrate. In contrast, the process of the present invention both allows for arranging the assay components by liquid handling operations only and for measurements without physical contact of an analytical sensor to the vesicles or cells while still regulating the gating activity namely by controlling the temperature change as a condition triggering the gating activity or by effective removal of a blocking agent to allow for the triggering by the temperature present or temperature change.

Further, the present invention offers the advantage of allowing to use a multitude of lipid membrane vesicles for analysis, which results in a statistically valuable measurement of a group of such vesicles, preferably eliminating side effects of single vesicle deviations or variations.

In the alternative to measuring the activity of an ion channel in respect of the permeation of cations, e.g. sodium, potassium of calcium ions, followed by a colorimetric of fluorescence detection of the change of the ion species permeated into the surrounding medium by its interaction with an additional dye, a direct colorimetric and/or fluorescence detection can be employed by preparing the lipid membrane vesicles to contain a dye or fluorescence compound in their inner volume, which dye of fluorescence compound is permeated by the ion channel integrated into the lipid membrane and therefore released into the surrounding medium. Again, a colorimetric or fluorescence detection is possible, identifying the rise in concentration of dye or fluorescent compound in the medium. A suitable fluorescent dye for use with e.g. potassium permeating ion channels is Lucifer Yellow, which can be detected at a wavelength of 535 nm after excitation by a wavelength of 485 nm.

A further advantage of the present invention is based on the use of lipid vesicles that are obtainable by synthetic generation from the constituent components, because the vesicles have pre-determined constituent compounds and a defined structure, i.e. vesicles are generated from defined membrane forming lipids, into which vesicles the isolated ion channel protein can be inserted by contacting the vesicles with the ion channel protein. Ion channel protein can be obtained by isolation from natural sources, e.g. from tissue or cultivated cells, and is preferably obtained by recombinant expression, e.g. in mammalian cell culture or by expression in bacteria or yeast and fungi, respectively, and isolated from the culture supernatant and/or from the expressing cells. The generation of lipid membrane vesicles having the ion channel incorporated into their membrane from isolated and defined constituent compounds in an *in vitro* generating process in contrast to whole cells allows to assign observed effects of the effector onto the gating activity directly, e.g. without interactions contributed by non-defined cellular components. Further, the process of the invention, as it does not depend on a direct physical or electrical contact of a measuring electrode or of an aperture made of an insulating material for separation of inner cell volume from surrounding medium, does not require high mechanical resistance of the vesicles to mechanical damage as e.g. patch clamp techniques do. In contrast, the contact-free triggering and contact-free measurement of gating activity of the invention allows to use lipid-membrane vesicles obtainable by *in vitro* generation from lipids in an aqueous medium, e.g. without natural cell walls because mechanical stress onto the ion channel containing vesicles is much reduced in comparison to patch-clamp techniques. Therefore, in relation to the invention, the lipid membrane can be embodied by a lipid bilayer, forming a vesicle in an aqueous medium.

Lipid membrane vesicles can be generated by resuspending a POPC lipid film in an aqueous 150 mM sodium chloride, 10 mM Tris-HCl solution at pH 8 and centrifugation at 60,000 rpm for 10 minutes at 4 °C, for example using a Beckman rotor 100.4. For incorporation of fluorescent dye, about 1.5 weight-% of the fluorescent dye can be incorporated into the aqueous solution, for example Lucifer Yellow. After centrifugation, the supernatant is removed and the vesicles are resuspended in 150 mM sodium chloride, 10 mM Tris, pH 8 and centrifuged for an additional 10 minutes. For incorporation of ion channel protein, isolated ion channel protein, e.g. obtained from recombinant expression, is solubilised in a mild detergent, e.g. in 30 mM octylglucoside, 150 mM sodium chloride, 10 mM Tris-HCl, pH 8 at a concentration of 10 to 1,000 µg/mL, preferably at about 100 µg/mL, and added to the lipid membrane vesicles (dry weight of about 10 mg/mL). For removal of the detergent or surfactant, the ion channel containing vesicles are dialysed, preferably for about 2 days against the same buffer, for example against a 250 -fold volume 150 mM sodium chloride, 10 mM Tris-HCl, pH 8 at 4 °C, including two to four changes of buffer.

Alternatively, lipid membrane vesicles can be generated from lipids comprised in the group of POPC (1-palmitoyl-2-arachidonoyl-sn-glycero-3-phosphocholine), POPG (1-palmitoyl-2-oleyl-sn-glycero-3-phospho-rac-(1-glycerole), POPE (1-palmitoyl-2-oleyl-sn-glycero-3-phosphocholine), preferably in a ratio of 3:1:1.

In the alternative to synthetically generated lipid membrane vesicles, plant cells, mammalian, bacterial or yeast and fungal cells can be used as a replacement, for example cultured cells that recombinantly express the ion channel localized in their cellular membrane and/or cell wall. In an alternative, *in vitro* translated proteins encoded by cRNA encoding the appropriate channel gene or heterologously expressed proteins can be used.

For the assay, 10 - 100 µL aliquots can be used and prepared for the assay by cooling on ice, followed by incubation at the changed temperature, e.g. by incubation for 5 min to change the temperature to the desired temperature. Alternatively, the energy necessary for a change of temperature can be introduced by irradiation with a laser pulse. In the case of a temperature change from a higher to a lower temperature, e.g. if the permeation permissive temperature is below body or ambient temperature, cooling can be effected by e.g. a thermostat, preferably a Peltier element as e.g. used in thermocyclers for PCR.

Due to the combination of advantages of the process of the invention, it is possible to adapt the process to a microtiter plate assay format, e.g. performing the process in a volume of 10 to 200 µL, preferably in about 100 µL. For manual and automated processing, the liquid preparations of lipid-membrane vesicles, preferably charged with the ion species or a dye permeating through the ion channel, and containing ion channel protein within the membrane in a suspension of an aqueous medium can be measured and transported by state-of-the-art liquid handling operations, e.g. pipetting. Optional addition of the blocking agent and of the effector under investigation can also be done by state-of-the-art liquid handling operations. For measurements, no physical contact of the lipid-membrane vesicles by sensors, e.g. electrodes is necessary. Triggering of gating activity is done by changing the temperature of the assay composition, preferably by using a thermostat, by laser irradiation and/or by ultrasonic vibration and/or addition of an effective removal agent, masking or complexing a blocking agent, i.e. contact free in respect of the lipid-membrane vesicles. Retrieval of measurement data is also done contact-free by colorimetric and/or fluorescence detection. As electrodes are not in direct contact with the vesicles, the alternative measurement method using electrodes for determination of a change of conductance and/or capacity is also contact-free in respect of the vesicles.

When preparing the lipid membrane vesicles to contain the specific ion species that is permeated by the ion channel protein, the ion species is preferably contained in the buffer used for preparing the lipid membrane vesicles, or introduced into the lipid membrane vesicles during a following dialysis. For colorimetric or fluorescence detection of the release of the ion species, e.g. potassium ions, from the lipid membrane vesicles into the surrounding medium, a dye specific for the ion species permeated through the ion channel is added to the surrounding medium. Permeation of the ion species from the lipid membrane vesicles into the surrounding medium will change the colorimetric absorbance and/or fluorescence properties of the dye, which can be detected by colorimetric and fluorescence detection, respectively. For detection of the release of fluorescent dye, an irradiation source emitting at the excitation wavelength is to be positioned to irradiate onto the sample, followed by detection of the emission at the emission wavelength by a detector, arranged in an angle above or below the sample to receive emitted light.

### Detailed description of the invention

The present invention is now described in greater detail with reference to the figures, wherein
- Figure 1 schematically shows an apparatus for performing the process of the invention,
- Figure 2 shows an SDS-PAGE gel with Coomassie blue staining,
- Figure 3 shows a graph of the relative fluorescence as a function of the time for a lipid membrane vesicle having ion channel protein integrated into its membrane (black, upper curve) in comparison a lipid membrane vesicle without ion channel protein (lower grey curve) with a heat pulse applied (arrow),
- Figure 4 shows a measurement of the relative fluorescence over time for samples of lipid membrane vesicles including an ion channel protein (black upper curve) in comparison to lipid membrane vesicles without ion channel protein (grey lower curve) at constant temperature conditions,
- Figure 5 shows fluorescence measurements indicating the triggering of gating activity at differing temperatures,
- Figure 6 shows the stability of fluorescence measurements after triggering gating activity,
- Figure 7 shows fluorescence measurements with triggering gating activity for differing concentrations of ion channel protein,
- Figure 8 shows a graph of the fluorescence measurements over the concentration of ion channel protein,
- Figure 9 shows fluorescence measurements with triggering gating activity in the presence of an effector influencing gating activity, and
- Figure 10 shows an analytical graph of the fluorescence measurements in the presence of an effector over the concentration of the effector.

The apparatus for performing the analytical process according to the invention comprises a vessel, e.g. in the form of a microtiter plate providing a plurality of wells, for receiving an aqueous medium containing the lipid membrane vesicles having an ion channel protein integrated into their membrane and containing in their inner volume an ion species or a dye that is permeated by the ion channel in its open state. For application of the temperature change, e.g. an increase in temperature from a lower temperature, e.g. from 0 to 5 °C up to an elevated temperature of 25, 37 of 42 °C, a thermostat can be comprised in the apparatus, or for reducing the temperature from an elevated level to cooling temperatures, respectively. As the application of a temperature change can, at least for a plurality of ion channel proteins, be replaced by the application of ultrasonic vibration, the apparatus is preferably equipped with an ultrasound generator, to transmit ultrasound vibrations to the vessels containing the vesicles, in addition to or in alternative to a thermostat.

As a further alternative to a thermostat, the temperature change from cooling to elevated temperatures can obtained by the application of thermal energy by microwave irradiation. Accordingly, the apparatus can also be equipped with a microwave generator capable of transmitting thermal energy to the vessel.

Further, for measurement of a change of electrical properties of the aqueous medium in which the vesicles are comprised, the apparatus can be equipped with a pair of electrodes introduceable into the aqueous medium, and connected to a device for measurement of a change of electric conductivity and/or electric capacity. In the case of detecting a change of electric conductivity and/or electric capacity of the medium for the determination of ion permeation, in this embodiment it is preferred that the aqueous medium containing the lipid membrane vesicles is essentially free of electrically conductive ion species.

As it is preferred for detection to use colorimetric and/or fluorescence measurement, the apparatus is preferably equipped with a light source irradiating the vessels and an optical detector, for example in the form of a CCD camera, or with an excitation light source at the excitation wavelength of the fluorescent dye and an optical detector for fluorescence measurement, respectively.

In a preferred embodiment, the change of temperature from cooling temperatures to elevated temperatures is generated by the introduction of thermal energy using a laser pulse. Accordingly, the apparatus is preferably equipped with a laser capable of introducing thermal energy into the solution contained in the vessels.

Further, it is preferred that the apparatus is equipped with a computing device capable of correlating signals detected during the change of temperature and/or before and after addition of an agent effectively removing a blocking agent, preferably correlating at least two measurement signals obtained from the vessels under different conditions. Measurement signals to be correlated can e.g. be obtained from vessels with different amounts of thermal energy introduced, from vessels incubated at different temperatures, and/or from vessels containing different concentrations of effector.

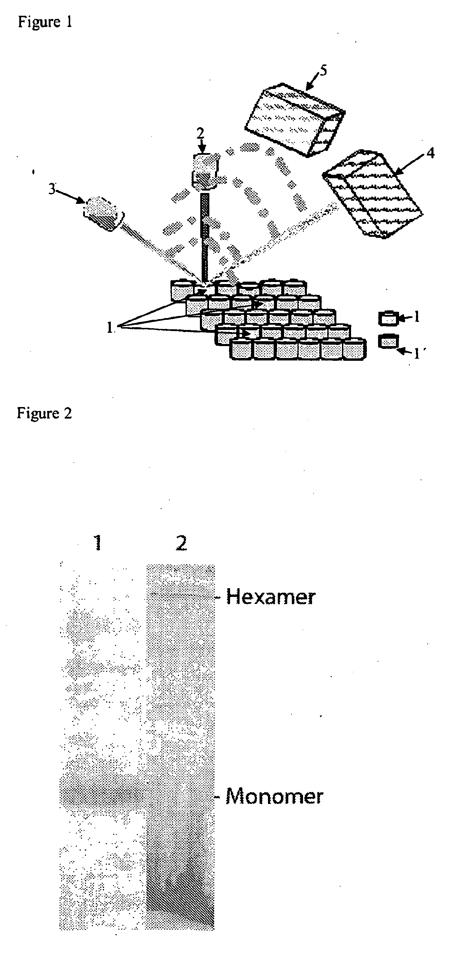

In Figure 1, an apparatus for performing the process of the invention is schematically depicted, showing vessels 1 in the form of a microtiter plate for receiving the aqueous medium containing the lipid membrane vesicles, wherein assay compositions with an effector at sufficient concentration to affect gating activity are indicated by vessels 1, whereas blank vessels indicated by 1' contain assay compositions without effective effector concentration.

According to the preferred embodiment of the apparatus, the change of temperature is realized by elevating the temperature of the sample by introduction of thermal energy, either by irradiation, e.g. by microwave or laser irradiation, or permeation is triggered by introduction of ultrasound by a generator for ultrasonic vibration, respectively. For detection of a change of fluorescence, an excitation light source 3 irradiates the sample in vessel 1 while a detector 4, e.g. a fluorescence detector or a CCD camera, is arranged to receive emitted light. For a determination of change of temperature, a thermal detector 5, e.g. an infrared camera, can further be arranged to receive thermal irradiation from vessel 1. During the process of analysis, irradiation source 3 concurrently irradiates sample vessel 1 that is subjected to the temperature change by irradiation or ultrasound from the generator 2 with concurrent measurement by the detector 4.

### Comparative Example 1: Temperature induced permeation of ion channel protein contained in lipid membrane vesicles

Lipid membrane vesicles are generated by resuspending a POPC lipid film in 150 mM sodium chloride, 10 mM Tris-HCl, pH 8, containing Lucifer Yellow as a replacement for the cation species permeated by the exemplary ion channel protein.

Following centrifugation, the supernatant is removed and vesicles are suspended in the same medium for 10 minutes. As the ion channel protein, connexin hCx26, reconstituted to its hexameric form after expression in the *E.coli* strain Top10 (Invitrogen), using the expression plasmid pTrcHisTOPO (available from Invitrogen, Germany) containing the hCx26 gene under the control of a trc-promoter. The gene sequence is accessible at GenBank under accession number NP 003995. Cultivation was in LB-medium containing ampicillin as the selection marker at 37 °C.

Monomeric ion channel protein is shown in lane 1 of Figure 2 in a denaturing SDS-PAGE, whereas lane 2 shows that after reconstitution in the presence of POPC-lipid, the hexamer is stable in SDS-PAGE, migrating at approximately 179 kDa.

For incorporation of ion channel protein, 250 µg/mL were solubilised in 30 mM octylglucoside, 150 mM sodium chloride, 10 mM Tris-HCl, pH 8 and added to 1 mL lipid membrane vesicle suspension (10 mg POPC/mL). After incubation for 1 to 12 hours under mild agitation at 4 °C, detergent was removed by dialysis for two days at 4 °C in 150 mM sodium chloride, 10 mM Tris-HCl, pH 8, including three changes of buffer volume. Finally, the vesicles were transferred by dialysis against buffer into 150 mM sodium chloride, 10 mM Tris-HCl, pH 8 at 4 °C for removal of the ion species, namely the replacement Lucifer Yellow or the physiologically permeated ion potassium, which are permeated by the ion channel protein present.

For closing of channels, calcium²⁺ ions could optionally be added to a final concentration of 1 to 100 µM, e.g. 50 µM for blocking ion channels, as the exemplary ion channel protein is Ca²⁺ sensitive, i.e. gating activity can be restored by removal of Ca²⁺.

In case, calcium was added to the vesicle preparation as a blocking agent, an equal or excess amount of EDTA, e.g. 10-25 mM was added for masking of the blocking agent in order to trigger permeation in dependence on temperature only.

For measuring, aliquots of 10 - 20 µL vesicle suspension, corresponding to 2.5 - 5 mg ion channel protein per reaction were pipetted into wells of a microtiter plate, which preferably is black for ease of detection.

In general, the buffer medium used for the generation of lipid membrane vesicles has to be suited for maintaining ion channel protein intact, i.e. having a pH in a range that does not impair ion channel function and free from inhibitory compounds, except for a blocking agent that can be added optionally and be removed or masked prior to performing the assay. Apart from control wells, the effector under investigation was added to wells at varying concentrations e.g. from 1 nM to 10 µM final concentration, using the inhibitor carbenoxolone as an exemplary effector compound.

Starting at a basic temperature of 4 °C, the microtiter plate was heated incrementally by steps of 2 °C each using a thermostat, with intermediate or permanent measurement of fluorescence emission under irradiation at the excitation wavelength.

Further, in the alternative to using Lucifer Yellow in the solution for preparing lipid membrane vesicles, in the case of the connexin hCx26 ion channel, potassium or sodium ions were incorporated into the inner volume solution of the vesicles for indirect fluorescence measurement of ion permeation. Otherwise, the same protocol as above was used, except that for detection, a fluorescent dye specific for potassium was included in the samples.

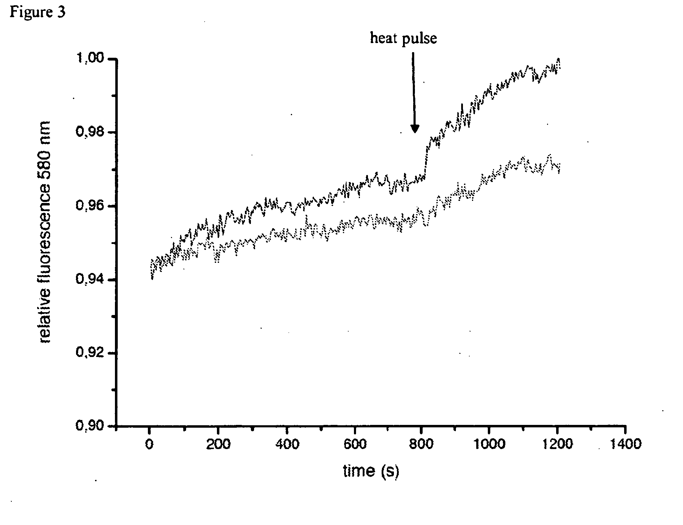

In Figure 3, measurement results of fluorescence of Lucifer Yellow (LY) at 580 nm is shown, with a heat pulse generated by constant rate heating or, alternatively, a heat pulse generated by laser irradiation applied at the time indicated by the arrow. The results show that before the application of the heat pulse, an increase in fluorescence can be observed, with a dramatic increase following the rise in temperature, which increase in fluorescence is significantly higher for ion channel protein containing vesicles (black upper curve) in comparison to a control assay of vesicles without ion channel protein (grey lower curve).

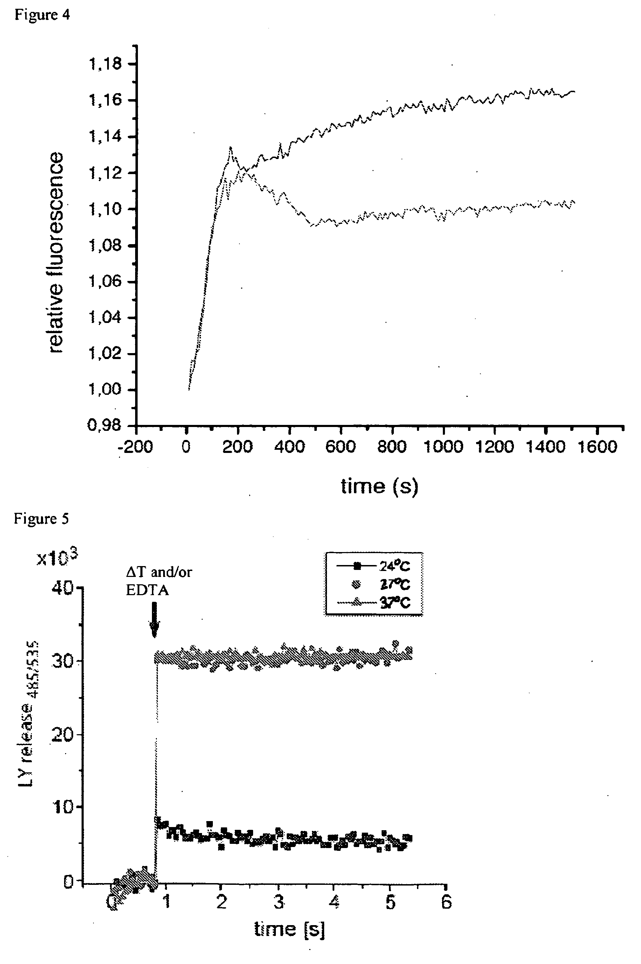

In Figure 4, the course of the fluorescence is shown in wells kept at a temperature above the temperature, at which permeation through the ion channel occurs for vesicles including the ion channel protein in their lipid membrane (upper black curve) in comparison to control vesicles, containing no ion channel protein (lower grey curve). This comparison demonstrates that the increase in fluorescence is actually due to the presence of ion channel protein, i.e. is not caused by release of Lucifer Yellow across the lipid membrane.

Using the Lucifer Yellow containing lipid vesicles with the ion channel protein connexin hCx26, gating activity was triggered by addition of EDTA at varying temperatures. In Figure 5 (lower curve of black boxes show 24 °C, upper curves show circles for 27 °C and triangles for 37 °C) it is shown that the elevation of the temperature to above 24 °C allows triggering permeation of Lucifer Yellow by removal of blocking agent (addition of EDTA at arrow), and higher temperatures of 27 and 37 °C do not lead to a significant increase in dye release. The signal remains stable for at least another 5 s. Addition of further EDTA does not cause activation of permeation at lower temperatures. As the experimental setup includes keeping the temperature constant, temperature differences between different wells should be kept at a minimum. The fluorescence measurement data from permeation fit the Boltzmann function with time constants of 20 ms at 23.7 °C, 12.2 ms at 27 °C and 13.5 ms at 37 °C.

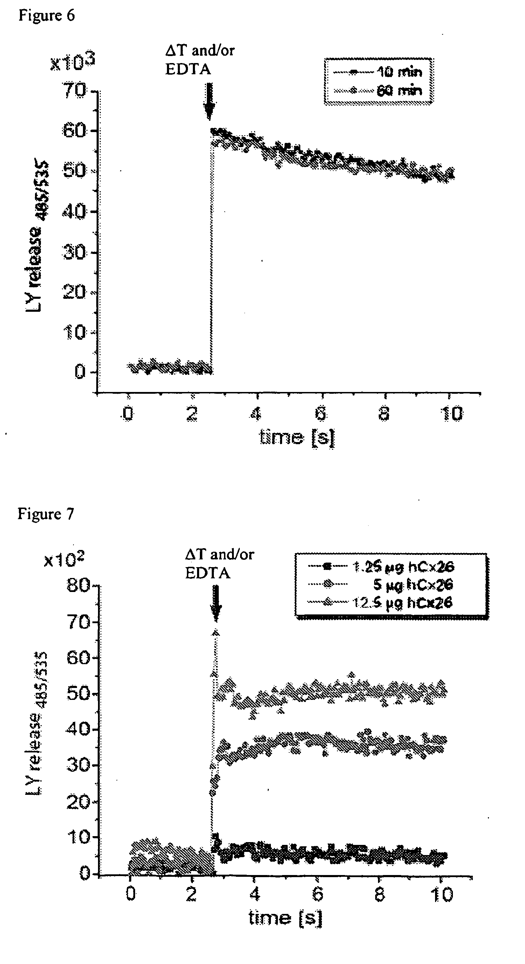

As shown in Figure 6, the fluorescence signal from dye release was stable for at least 10 to 60 min, indicating that stable signals for measurement can be obtained after a duration of the assay of 60 min.

In the alternative to removal of the blocking agent Ca²⁺ by addition of EDTA, a rise in temperature, e.g. by application of a laser pulse or ultrasonic vibration was used for triggering gating activity, i.e. permeation.

Preferably, the concentration of ion channel protein was adjusted to 0.25 to 5 µg/well, because it was found that for the present ion channel connexin hCx26 that concentration at below 0.25 µg/well, no signal of dye release could be detected, whereas concentrations above 5 µg/well caused unstable fluorescence signals. For triggering permeation activity, EDTA was added to a final concentration of 50 mM, or a heat pulse or ultrasonic vibration was applied. Results for different amounts of ion channel protein (hCx26, lowest curve of black boxes are 1.25 µg, middle curve of grey circles are 5 µg, upper curve of grey triangles are 1.25 µg) are shown in Figure 7, wherein the arrow indicates the triggering influence of removal of the blocking agent, increase in temperature, or ultrasonic vibration.

### Comparative Example 2: Influence of effectors on the temperature induced permeation of ion channel protein contained in lipid membrane vesicles

In general, the addition of an effector acting onto the ion channel leads to a change of the concentration of the ion species permeating from the vesicle into the surrounding aqueous medium and, hence, to an increase of the fluorescence generated from the fluorescent of the dye specific for the ion species. From a comparison of the increase of fluorescence at increasing temperatures of a reaction composition containing the effector to that measured for an identical composition, except for the absence of effector, allows to characterize the effector at its given concentration in the assay, e.g. as an inhibitor, activator or modulator of the gating activity. Before measurements, vesicles were adjusted to 50 µM Ca²⁺.

Keeping the temperature of the black microtiter plate constant at 28 °C, test substances were added to wells. As an exemplary ion channel inhibitor, carbenoxolone was added up to final concentrations of 10 mM to individual wells. For measurement, the microtiter plate could be kept at a temperature above which permeation could be triggered by addition of EDTA, and adding EDTA, i.e. by removal of the blocking agent Ca²⁺.

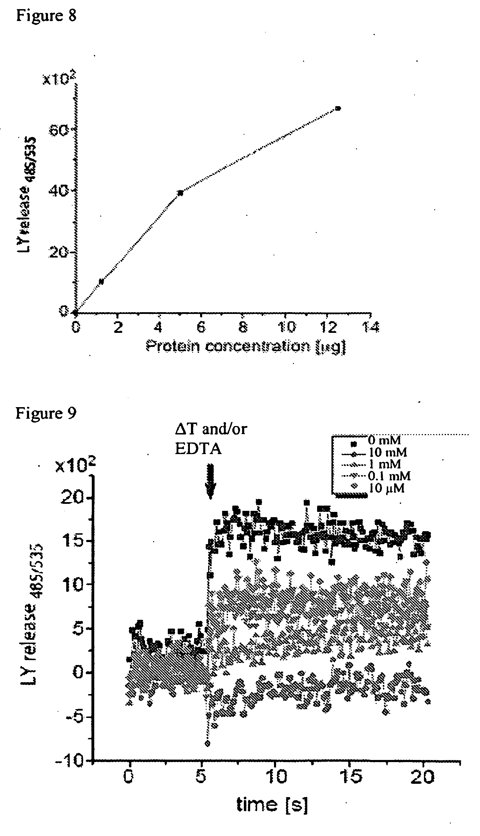

Alternatively, the microtiter plate was kept at a temperature below which permeation was triggered by removal of the blocking agent, e.g. at 5 to 10 °C in the absence of a blocking agent, and gating activity was triggered by applying a heat pulse, e.g. generated by laser irradiation. Triggering of gating activity by removal of the blocking agent or, preferably, by elevating the temperature, is indicated by the arrow in Figure 9. Concentrations of carbenoxolone are given in the inset, control (0 mM) is without inhibitor.

In the alternative to generating the elevation in temperature by application of a laser pulse, a controlled increase in temperature can be caused by a thermostat heating the assay solution.

Using the same experimental setup, the inhibitory effect of heptanol could be shown in the nanomolar to picomolar range, for cobalt ions in the millimolar to micromolar range.

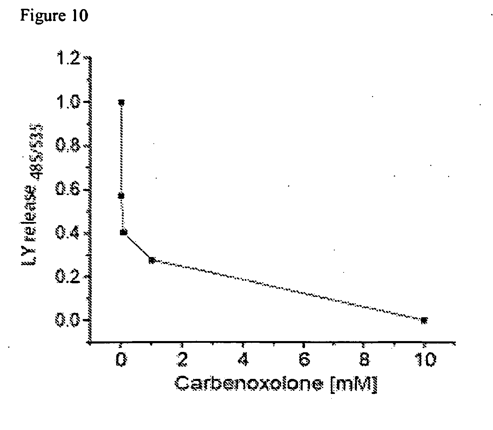

The concentration dependency of gating activity, i.e. the inhibitory effect of the effector carbenoxolone, is graphically analyzed in Figure 10.

In further experiments, the same effects could be shown for using ultrasonic vibration instead of a heat pulse and controlled heating, respectively.

### Comparative Example 3: Temperature-induced permeation of peptide through ion channel protein contained in lipid membrane vesicles

As representative for permeation of larger molecules than alkaline or alkaline earth ions, 8- to 10-mer synthetic peptides were chosen and flourescence-labelled by chemical linkage to Lucifer Yellow or to fluorescein. Following the general procedure of Example 1, purified connexins, e.g. Cx43, n Cx43 and further known connexins, including connexins of different families, were used to generate lipid-membrane vesicles containing in their inner volume the labelled peptide.

Permeation of labelled peptide from the inner volume of the lipid-membrane vesicle containing membrane-bound connexin was triggered by raising the temperature as described above.

However, lipid-membrane vesicles were preferably replaced by animal cells transfected with an expression cassette encoding the ion channel protein, e.g. nCx43, nCx26, nCx46. For introduction of labelled peptide, synthetic peptides containing predominantly or consisting of D-amino acids, labelled with Lucifer Yellow or fluorescein coupled to an N- or C-terminal lysine were used. Introduction into lipid membrane vesicles was done according to Example 1.

## Claims

1. Process for the detection of the influence of an effector compound on the activity of an ion channel protein, comprising the steps of
generating a lipid membrane vesicle in an aqueous medium in the presence of a gating activity specific blocking agent,
the vesicle containing in its lipid membrane an ion channel protein, which vesicle in its inner volume contains an ion species that is capable of permeation through the ion channel protein,
contacting the effector compound with the ion channel protein,
applying a temperature change to the vesicle,
effectively removing the blocking agent, and
subsequent detection of a change of the concentration of the ion species in the aqueous medium.

2. Process according to claim 1, **characterized in that** effectively removing the blocking agent for triggering permeation effectively is at or above the permissive temperature at which in the absence of blocking agent permeation would occur.

3. Process according to claim 1, **characterized in that** the permissive temperature is below body or ambient temperature and the temperature change is from a higher to a lower temperature.

4. Process according to claim 1, **characterized in that** effectively removing the blocking agent is prior to applying the temperature change.

5. Process according to claim 1, **characterized in that** applying a change of temperature during detection is replaced by incubating at least two aliquots of aqueous medium containing vesicles at different temperatures and comparing the changes of the concentration of ion species in the aqueous medium.

6. Process according to one of the preceding claims, **characterized in that** detection of the ion species is done by determination of the electric conductivity or electric capacity of the aqueous medium containing the vesicle.

7. Process according to one of claims 1 to 5, **characterized in that** detection of the change of concentration of ion species in the aqueous medium is done by addition of a dye to the aqueous medium, which dye is capable of indicating a change of concentration of the ion species by changing its colorimetric or fluorescence properties.

8. Process according to one of claims 1 to 5, **characterized in that** the ion species is replaced by a dye capable of permeation through the ion channel protein.

9. Process according to one of the preceding claims, **characterized in that** the ion species is a protein, a peptide, a nucleotide or a nucleic acid.

10. Process according to claim 9, **characterized in that** the protein, peptide or nucleic acid is labelled with a dye.

11. Process according to one of the preceding claims, **characterized in that** the dye is selected from colorimetrically and/or fluorescence detectable compounds selected from the group comprising Lucifer Yellow, SBFI, PBFI, CoroNa Green Na⁺ indicator, and CoroNa Red Na⁺ indicator.

12. Process according to one of the preceding claims, **characterized in that** the vesicles are contacted with a blocking agent and the blocking agent is effectively removed or masked prior to application of the change of temperature.

13. Process according to one of the preceding claims, **characterized in that** the temperature is changed by warming the vesicles by introduction of thermal energy.

14. Process according to one of the preceding claims, **characterized in that** the temperature is changed by introduction of thermal energy by application of laser and/or microwave irradiation onto the vesicles.

15. Process according to one of the preceding claims, **characterized in that** the change of temperature is replaced by the application of ultrasound.

16. Process according to one of the preceding claims, **characterized in that** it is performed in a volume of 5 to 200 µL total volume.

17. Use of an apparatus for performing a process according to one of the preceding claims, comprising a vessel (1) receiving lipid membrane vesicles containing ion channel protein in the membrane,
a generator (2) for generating thermal irradiation or ultrasound affecting the vessel (1),
an excitation light source (3) generating light of the wavelength exciting a fluorescent dye present in the vessel (1), and
a detector (4) for measuring irradiation emitted from the fluorescent dye upon excitation.

18. Use according to claim 17, **characterized in that** the apparatus comprises a calculator unit, capable of correlating at least two signals obtainable from the detector (4).

## Patentansprüche

1. Verfahren zum Nachweis des Einflusses eines Wirkstoffs auf die Aktivität eines lonenkanalproteins, das die Schritte
der Erzeugung eines Lipidmembran-Vesikels in einem wässrigen Medium in der Gegenwart eines spezifischen Blockierungsmittels der Öffnungs-Schließaktivität, wobei das Vesikel ein Ionenkanalprotein in seiner Lipidmembran enthält, welches Vesikel in seinem Innenvolumen eine Ionenart enthält, die zur Permeation durch das Ionenkanalprotein in der Lage ist,
des Kontaktierens des Wirkstoffs mit dem Ionenkanalprotein,
des Aufbringens einer Temperaturänderung auf das Visikel,
des wirksamen Entfernens des Blockierungsmittels und
der nachfolgenden Detektion einer Änderung der Konzentration der Ionenart in dem wässrigen Medium umfasst.

2. Verfahren nach Anspruch 1, **dadurch gekennzeichnet, dass** das wirksame Entfernen des Blockierungsmittels zur wirksamen Auslösung der Permeation bei oder oberhalb der permesiven Temperatur wirksam ist, bei welcher in Abwesenheit eines Blockierungsmittels die Permeation auftreten würde.

3. Verfahren nach Anspruch 1, **dadurch gekennzeichnet, dass** die permissive Temperatur unterhalb der Körpertemperatur oder Umgebungstemperatur ist und die Temperaturänderung von einer höheren zu einer niedrigen Temperatur erfolgt.

4. Verfahren nach Anspruch 1, **dadurch gekennzeichnet, dass** das wirksame Entfernen des Blockierungsmittels vor dem Aufbringen der Temperaturänderung erfolgt.

5. Verfahren nach Anspruch 1, **dadurch gekennzeichnet, dass** das Aufbringen einer Temperaturänderung während der Detektion durch Inkubieren von wenigstens zwei Aliquots des wässrigen Mediums, das Vesikel enthält, bei verschiedenen Temperaturen und das Vergleichen der Änderungen der Konzentration der Ionenart in dem wässrigen Medium ersetzt wird.

6. Verfahren nach einem der voranstehenden Ansprüche, **dadurch gekennzeichnet, dass** die Detektion der Ionenart durch Bestimmung der elektrischen Leitfähigkeit oder der elektrischen Kapazität des wässrigen Mediums erfolgt, das das Vesikel enthält.

7. Verfahren nach einem der Ansprüche 1bis 5, **dadurch gekennzeichnet, dass** die Detektion der Änderung der Konzentration der Ionenart in dem wässrigen Medium durch Zugabe eines Farbstoffs in das wässrige Medium erfolgt, wobei der Farbstoff in der Lage ist, eine Änderung der Konzentration der Ionenart durch Ändern seiner kolorimetrischen oder Fluorenszenz-Eigenschaften anzuzeigen.

8. Verfahren nach einem der Ansprüche 1 bis 5, **dadurch gekennzeichnet, dass** die Ionenart durch einen Farbstoff ersetzt wird, der zur Permeation durch das Ionenkanalprotein in der Lage ist.

9. Verfahren nach einem der voranstehenden Ansprüche, **dadurch gekennzeichnet, dass** die Ionenart ein Protein, ein Peptid, ein Nukleotid oder eine Nukleinsäure ist.

10. Verfahren nach Anspruch 9, **dadurch gekennzeichnet, dass** das Protein, Peptid oder die Nukleinsäure mit einem Farbstoff markiert ist.

11. Verfahren nach einem der voranstehenden Ansprüche, **dadurch gekennzeichnet, dass** der Farbstoff aus kolorimetrisch und/oder mittels Fluoreszenz detektierbarer Verbindungen ausgewählt ist, die aus der Gruppe ausgewählt sind, die Lucifer, Yellow, SBFI, PBFI, CoroNa Green Na⁺ Indikator und CoroNa Red Na⁺ Indikator, umfasst.

12. Verfahren nach einem der voranstehenden Ansprüche, **dadurch gekennzeichnet, dass** die Vesikel mit einem Blockierungsmittel kontaktiert werden und das Blockierungsmittel vor Aufbringung der Temperaturänderung wirksam entfernt oder maskiert wird.

13. Verfahren nach einem der voranstehenden Ansprüche, **dadurch gekennzeichnet, dass** die Temperatur durch Erwärmen der Vesikel durch Einführung thermischer Energie verändert wird.

14. Verfahren nach einem der voranstehenden Ansprüche, **dadurch gekennzeichnet, dass** die Temperatur durch Einführung von thermischer Energie durch Aufbringung von Laser- und/oder Mikrowellenstrahlung auf die Vesikel verändert wird.

15. Verfahren nach einem der voranstehenden Ansprüche, **dadurch gekennzeichnet, dass** die Änderung der Temperatur durch die Aufbringung von Ultraschall ersetzt wird.

16. Verfahren nach einem der voranstehenden Ansprüche, **dadurch gekennzeichnet, dass** es in einem Volumen von 5 bis 200 µL Gesamtvolumen durchgeführt wird.

17. Verwendung eines Geräts zur Durchführung eines Verfahrens nach einem der voranstehenden Ansprüche, das einen Behälter (1), der Lipidmembran-Vesikel aufnimmt, die in der Membran ein Ionenkanalprotein enthalten,
einen Generator (2) zur Erzeugung von auf das Gefäß (1) einwirkender thermischer Strahlung oder Ultraschall,
eine Anregungslichtquelle (3), die Licht der Wellenlänge zur Anregung eines in dem Gefäß (1) anwesenden Fluoreszenzfarbstoffs erzeugt und
einen Detektor (4) zur Messung von Strahlung, die bei Anregung von dem Fluoreszenzfarbstoff emittiert wird, umfasst.

18. Verwendung nach Anspruch 17, **dadurch gekennzeichnet, dass** das Gerät eine Rechnereinheit umfasst, die in der Lage ist, wenigstens zwei von dem Detektor (4) erhältliche Signale zu korrelieren.

## Revendications

1. Procédé destiné à détecter l'influence d'un agent actif sur l'activité d'une protéine de canal ionique, comprenant les étapes de
générer une vésicule membranaire lipide dans un fluide aqueux en présence d'un agent spécifique bloquant l'activité d'ouverture et de fermeture,
sachant que la vésicule contient dans sa membrane lipide une protéine de canal ionique, laquelle vésicule contient dans son volume intérieur une espèce d'ion capable de perméation à travers la protéine de canal ionique,
contactant l'agent actif avec la protéine de canal ionique
appliquant un changement de température à la vésicule,
en enlevant effectivement l'agent bloquant, en
détectant de façon consécutive un changement de la concentration de l'espèce d'ion dans le fluide aqueux.

2. Procédé selon la revendication 1, **caractérisé par le fait que** l'enlèvement de l'agent bloquant pour déclencher la perméation est effectivement à ou au-dessus de la température admise à laquelle l'absence de perméation de l'agent bloquant se produirait.

3. Procédé selon la revendication 1, **caractérisé par le fait que** la température admise est en dessous de la température du corps ou de la température ambiante et que le changement de température se fait à partir d'une température supérieure à une température inférieure.

4. Procédé selon la revendication 1, **caractérisé par le fait que** l'enlèvement de l'agent bloquant se fait avant d'appliquer le changement de température.

5. Procédé selon la revendication 1, **caractérisé par le fait que** l'application d'un changement de température pendant la détection est remplacée par l'incubation d'au moins deux aliquots de fluide aqueux contenant des vésicules à différentes températures et la comparaison des changements de concentration de l'espèce d'ion dans le fluide aqueux.

6. Procédé selon une des revendications précédentes, **caractérisé par le fait que** la détection de l'espèce d'ion se fait en déterminant la conductivité électrique ou la capacité électrique du fluide aqueux contenant la vésicule.

7. Procédé selon une des revendications 1 à 5, **caractérisé par le fait que** la détection du changement de concentration de l'espèce d'ion dans le fluide aqueux se fait en ajoutant un colorant au fluide aqueux, lequel colorant est capable d'indiquer un changement de concentration de l'espèce d'ion en changeant ses propriétés colorimétriques ou de fluorescence.

8. Procédé selon une des revendications 1 à 5, **caractérisé par le fait que** l'espèce d'ion est remplacée par un colorant capable de perméation à travers la protéine de canal ionique.

9. Procédé selon une des revendications précédentes, **caractérisé par le fait que** l'espèce d'ion est une protéine, un peptide, un nucléotide ou un acide nucléique.

10. Procédé selon la revendication 9, **caractérisé par le fait que** la protéine, le peptide ou l'acide nucléique est marqué par un colorant.

11. Procédé selon une des revendications précédentes, **caractérisé par le fait que** le colorant est sélectionné à partir de composés détectables au niveau de la colorimétrie et/ou de la fluorescence à partir du groupe comprenant l'indicateur Lucifer Yellow, SBFI, PBFI, CoroNa Green Na⁺, et l'indicateur CoroNa Rouge Na⁺

12. Procédé selon une des revendications précédentes, **caractérisé par le fait que** les vésicules sont contactées par un agent bloquant et que l'agent bloquant est effectivement enlevé ou masqué avant l'application du changement de température.

13. Procédé selon une des revendications précédentes, **caractérisé par le fait que** la température est changée en réchauffant les vésicules en introduisant de l'énergie thermique.

14. Procédé selon une des revendications précédentes, **caractérisé par le fait que** la température est changée en introduisant de l'énergie thermique en appliquant une irradiation laser et/ou microonde sur les vésicules.

15. Procédé selon une des revendications précédentes, **caractérisé par le fait que** le changement de température est remplacé en appliquant un ultrason.

16. Procédé selon une des revendications précédentes, **caractérisé par le fait qu'**il est effectué dans un volume de 5 à 200 µL de volume total.

17. Utilisation d'un appareil afin d'effectuer un procédé selon une des revendications précédentes, comprenant un récipient (1) recevant des vésicules membranaires lipides contenant une protéine de canal ionique dans la membrane,
un générateur (2) destiné à générer une irradiation thermique d'ultrason affectant le récipient (1), une source lumineuse excitante (3) générant une lumière d'une longueur d'onde excitant le colorant fluorescente présente dans le récipient (1), et un détecteur (4) destiné à mesurer l'irradiation émise par le colorant sous l'effet de l'excitation.

18. Utilisation selon la revendication 17, **caractérisée par le fait que** l'appareil comprend une unité calculatrice capable de corréler au moins deux signaux pouvant être obtenus du détecteur (4).
